# EUROPEAN PATENT SPECIFICATION

(11) **EP 0 873 739 B1**
(45) Date of publication and mention of the grant of the patent: **07.04.2004**
(21) Application number: 97106669.1
(22) Date of filing: 22.04.1997
(51) Int. Cl.: A61F 13/56, A61F 13/58

(54) **Absorbent article comprising dual fixation means of the mechanical and of the body contact adhesive type**
Saugfähiger Artikel mit Doppelverschluss, bei dem eine mechanische Befestigung mit einer Klebebefestigung am Körper kombiniert ist
Article absorbant pourvu d'un double moyen de fixation comportant une fixation mécanique et une fixation par adhésif sur le corps

(43) Date of publication of application: 28.10.1998
(73) Proprietor: THE PROCTER & GAMBLE COMPANY, Cincinnati, Ohio 45202 (US)
(72) Inventor: Thurnay, Eva Susanne Dominique, 60489 Frankfurt (DE); Ramos, Agustin, 61440 Oberursel (DE); Schöne, Rainer, 61462 Königstein/Ts (DE); Coles, Peter, 66023 Francavilla al Mare, Chieti (IT); Cinelli, Fabio, 40133 Bologna (IT)
(74) Representative: Canonici, Jean-Jacques

(56) References cited:
- DE-U- 9 001 834
- GB-A- 2 284 767
- US-A- 5 114 419

## Description

### General field of the invention

This invention is directed to hygienic absorbent articles, such as diapers, adult incontinence articles, feminine protection articles and the like.

### Background / Prior art

Absorbent articles are well known in the art. These articles typically have an absorbent core, which is held or positioned against the body of the wearer during use by a fixation means, such that the bodily exudates are caught by the article.

Such fixation means can be integral with the article. Thereby, the fixation means can be attachment means on front and back waist regions, which are fixed such as by mechanical engagement means or adhesive tape means to each other to then encircle the waist of the wearer. In a further modification of this design, elastic elements can be designed so as to enhance the comfort for the wearer without risking slippage of the article during movements. U.S. 5,066,289 discloses a disposable diaper with an outer fastening means comprising least an adhesive fastening tab and an associate release tab and an inner fastening means comprising a mechanical fastener. In an even further design variant, the front and back waist portion can be permanently fixed, whilst application of the article and fixation during use are achieved by elastication only, e.g. in the upper waist region of the article as well known from undergarments.

Also widespread is the application of affixing the absorbent pad to the undergarment of the wearer, such as by adhesive means, often called "panty fastening adhesive".

Such fixation means can also be external to the absorbent body, such as being exemplified by "pad/pant" systems, whereby for example a specially designed elastic pant is worn which fixes the absorbent body, such as described in EP 0811362 A.

The above fixation means all rely on frictional forces between the skin of the wearer and the article and can therefore also be referred to as "mechanical fixation means". An alternative to this principle has for example been described in EP 850 619 A, or GB - A - 2.284.767 relating to the use of topical adhesive attachment of the absorbent articles to the skin of the wearer often referred to as "body adhesives". These benefits have been described in particular for feminine hygiene applications, and other applications without heavy loading of exudates.

U.S. 5,114,419 discloses a hygienic device, with a self-adhesive material located at its edges or coated to be adherent to the user's body excluding the penetration of pathogens or irritants from contaminating environments.

However, all of the described variants still carry the need for further improvement. For example, the articles which are kept on the body by frictional forces still can loose the registry with wearers body openings, resulting in poor performance and/or discomfort for the wearer

The topical adhesives improve positioning of the article, however, in case of increased weight of the article, such as when being loaded during use, in particular with urine and/or faeces, these articles cannot be hold in place without the risk of too high straining of the skin of the wearer.

All prior art systems being based upon the mechanical fixation means further have the opportunity for improvements in the area of application of the article to the wearer. Be this for babies or be this for handicapped or disabled incontinent adults, in both cases a helper such as a parent or a caretaker, have to apply the absorbent article underneath the wearer, generally requiring difficult and delicate handling. Also, when the user is applying the article him/herself, there is still a need for easing application, especially for articles with relatively large dimensions such as adult incontinence pads, or for accurate positioning such as for feminine hygiene articles.

### Objects of the invention

Henceforth, it is an object of the present invention to provide absorbent articles with improved fit and sealing by combining the secondary topical adhesive fixation means with other primary fixation means, so as to exploit the synergy of the combination of the two features.

It is a further object of the invention to improve on sustained fit during use.

It is an even further object of the present invention to exploit the benefits of this combination during application of the articles, whereby special areas of the article with an topical adhesive can be easily applied to the wearer, whereas the primary fastening means can then be easily and accurately applied. ,

It is an even further object of the present invention, to provide such absorbent articles with improved means for disposability, whereby the topical adhesive fixation means can further ease clean and hygienic disposal of the soiled article after use.

### Summary

The invention is concerned with absorbent articles such as adult incontinence articles, baby diapers or feminine hygiene pads having a dual fixation system. Therein, a primary fixation means which can be of the adhesive tape type, or be a hook/loop system to combine the initially separated front and rear waist regions of an absorbent article, or can be an elasticised belt system being unitary with the absorbent member, such as in articles of the pant style. The secondary fixation means is of the body adhesive type specified in claim 1, thereby removably attaching selected regions of the absorbent article to the body of the wearer.

### Brief description of drawings

Fig. 1 is showing an exemplifying absorbent article known from the prior art, in three specific views.
Fig. 2 shows exemplified application of body adhesives to an article, such as adult incontinence pad.

### Detailed description

As used herein, the term "absorbent articles" refers to devices which absorb and contain body exudates, and more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner).

An absorbent article generally at least comprises
- an absorbent core (which may consist of sub-structures);
- a fluid pervious topsheet;
- chassis elements for sealing the body exudates for example from the clothing of the wearer, such as a backsheets or elastication features;
- fixation means to support the article during use, i.e. to establish and maintain its registry with exudate releasing body openings.

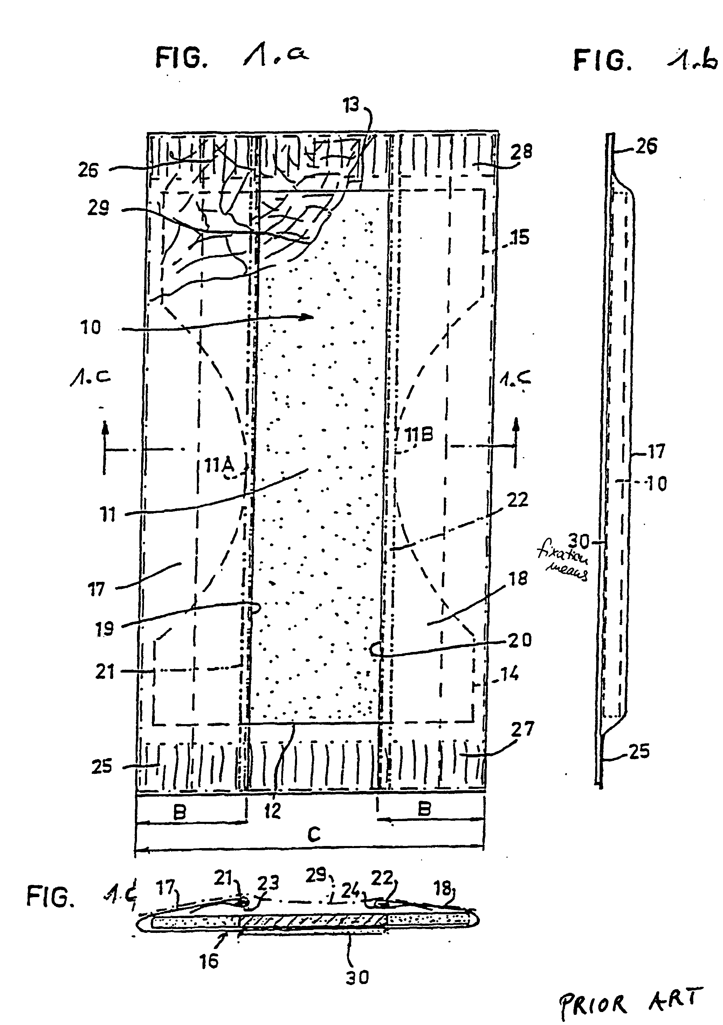

In Fig. 1 a, b, and c a prior art absorbent article is exemplified, which could be a baby diaper or an adult incontinence pad.

The article comprises an absorbent core 10, designed for absorbing and containing fluids, in most instances primarily aqueous based. The absorbent core 10 may be any absorbent means which is generally compressible, conformable, and capable of absorbing and retaining primarily aqueous liquids. Examples of suitable absorbent materials include comminuted wood pulp, creped cellulose wadding; meltblown polymers; chemically stiffened, modified or cross-linked cellulosic fibres; tissue including tissue wraps and tissue laminates; absorbent foams; absorbent sponges; superabsorbent polymers; absorbent gelling materials; or any equivalent material or combinations of materials. The configuration and construction of the absorbent pad may also be varied (e.g., the absorbent pad may have varying caliper zones, a hydrophilic gradient, a superabsorbent gradient, or lower average density and lower average basis weight acquisition zones; or may comprise one or more layers or structures).

Exemplary absorbent structures for use as absorbent pad as used in the disposable industry are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,888,231 entitled "Absorbent Core Having A Dusting Layer" issued to Angstadt on December 19, 1989; and U.S. Patent 4,834,735, entitled "High Density Absorbent Members Having Lower Density and Lower Basis Weight Acquisition Zones", issued to Alemany et al. on May 30, 1989. Other absorbent pad designs are described in EP 0631768 A and EP 0640330 A.

US patent no 4,411,660 discloses in an absorbent product two layers of absorbent material of different types, such that the upper layer gels slower than the first layer.

European Patent Specification EP-B-0 401 189 discloses that favourable properties of absorbent products can be achieved by using two different types of absorbent gelling material in separate layers, rather than as a mixture of the two absorbent gelling materials in a single layer.

The hydrogel-forming absorbent polymers useful in the present invention include a variety of substantially water-insoluble, but water-swellable polymers capable of absorbing large quantities of liquids. Such polymers materials are also commonly referred to as "hydrocolloids", or "superabsorbent" materials. These hydrogel-forming absorbent polymers preferably have a multiplicity of anionic, functional groups, such as sulfonic acid, and more typically carboxy, groups. Examples of polymers suitable for use herein include those which are prepared from polymerizable, unsaturated, acid-containing monomers. Thus, such monomers include the olefinically unsaturated acids and anhydrides that contain at least one carbon to carbon olefinic double bond. More specifically, these monomers can be selected from olefinically unsaturated carboxylic acids and acid anhydrides, olefinically unsaturated sulfonic acids, and mixtures thereof.

As described above, the hydrogel-forming absorbent polymers are preferably slightly network crosslinked. Network crosslinking serves to render the polymer substantially water-insoluble and, in part, determines the absorptive capacity and extractable polymer content characteristics of the precursor particles and the resultant macrostructures. Processes for network crosslinking the polymers and typical network crosslinking agents are described in greater detail in the herein before-referenced U.S. Patent 4,076,663, and in DE-A-4020780 (Dahmen).

In order to be able to compare absorbent articles for varying end use conditions, or differently sized articles, the "ultimate storage capacity" has been found to be a suitable measure.

For example, babies are representing a typical usage group, but even within this group the amount of urine loading, frequency of loading, composition of the urine will vary widely from smaller babies (new-born babies) to toddlers on one side, but also for example among various individual toddlers.

Another user group may be larger children, still suffering from a certain form of incontinence.

Also, incontinent adults can use such articles, again with a wide range of loading conditions, generally referred to as light incontinence ranging up to severe incontinence.

Henceforth, absorbent articles being able to cope with such requirements should have the capability of picking up such amounts of urine. These amounts of fluids have to be absorbed by materials which can ultimately store the bodily fluids, or at least the aqueous parts of these, such that - if any - only little fluid is left on the surface of the article towards the wearers skin. The term "ultimate" refers in one respect to the situation as in the absorbent article at long wearing times, in the other respect to absorbent materials which reach their "ultimate" capacity when being equilibrated with their environment. This can be in such an absorbent article under real in-use conditions after long wearing times, or this also can be in a test procedure for pure materials or material composites. As many of the processes under consideration have asymptotic kinetic behaviour, one skilled in the art will readily consider "ultimate" capacities to be reached when the actual capacity has reached a value sufficiently close to the asymptotic endpoint, e.g. relative to the equipment measurement accuracy.

As an absorbent article can comprise materials which are primarily designed to ultimately store fluids, and other materials which are primarily designed to fulfil other functions such as acquisition and/or distribution of the fluid, but may still have a certain ultimate storage capability, suitable core materials according to the present invention are described without attempting to artificially separate such functions. Nonetheless, the ultimate storage capacity can be determined for the total absorbent core, for regions thereof, for absorbent structures, or even sub-structures, but also for materials as being used in any of the previous.

As discussed in the above for varying the dimensions of the article, one skilled in the art will be able to readily adopt the appropriate ultimate storage capacities for other intended user groups. For example, Adult Incontinence articles intended for use with severly incontinent persons can contain 9 g of superabsorbent material having an absorbent capacity of about 31 ml/g when submitted to the well known Teabag centrifuge capacity test, and 97 g of conventional cellulosic airfelt having a capacity of about 4 ml/g, thus resulting in a total ultimate storage capacity of about 667 ml. Other examples relate to articles for light incontinent persons. For example ATTENDS MINI has an ultimate storage capacity of about 70 ml, ATTENDS MINI PLUS of 90 ml, or ATTENDS NORMAL of about 167 ml, with all these products sold by Procter & Gamble in various countries in Europe.

Referring again to Figures 1, said absorbent core has waist regions 14, 15 next to the longitudinal edges 12, 13, and a crotch region 11 connecting these regions 14, 15, which has a minimum width "A". This width "A" will be the result of optimising the fluid handling requirements in this crotch region with the dimensions of the wearer. As exemplified in Fig. 1 a, the width in the waist region 14, 15 is about twice the width of the crotch region 11.

The absorbent core 10 is connected with a fluid impervious backsheet 16, such as a conventional liquid impervious. The backsheet 16 may thus comprise a woven or nonwoven material, polymeric films such as thermoplastic films of polyethylene or polypropylene, or composite materials such as a film-coated nonwoven material. Often, the backsheet is a thermoplastic film having a thickness of from about 0.012 mm (0.5 mil) to about 0.051 mm (2.0 mils), such as a blown or cast PE film as available under the dsignation RR8220 (blown films) and RR5475 (cast films) as manufactured by Tredegar Industries, Inc. of Terre Haute, IN. Such a backsheet 16 is preferably embossed and/or matte finished to provide a more clothlike appearance.

Further, the backsheet 16 may permit vapours to pass through while still preventing liquids from penetrating through the backsheet 16.

Backsheet 16 may have both width and length dimensions exceeding the dimensions of the absorbent core 10, thereby forming peripheral edges. The region of the backsheet 16 which extend outwardly of the core in longitudinal direction is referred to as endflaps.

The relatively wide lateral edges 17, 18 of the backsheet 16 are folded inwardly so as to at least partially overlaying the absorbent core 10. The free longitudinal edges 19, 20 comprise an elastication feature 21, 22, such as an elastic band or stripe. When the absorbent article is stretched out flat as indicated in Fig. 1 these elastic features are stretched so as to allow contraction for better body conformity during use.

The lateral edges 17, 18 are now in a tubular arrangement 23, 24 with the unfolded parts, whereby the stretched elastics which are connected at their ends with the edges 25, 26 respectively 27, 28 of the inwardly folded lateral edges 17, 18.

A topsheet 29 is attached to the inwardly folded lateral edges 17, 18 oriented towards the wearer. The topsheet 29 is compliant, soft feeling. Further, the topsheet 29 is liquid pervious permitting fluids like urine liquids to readily penetrate through its thickness. A suitable topsheet may be manufactured from a wide range of materials, such as porous foams; reticulated foams; apertured plastic films; or woven or nonwoven webs of natural fibres (e.g., wood or cotton fibres), synthetic fibres (e.g., polyester or polypropylene fibres), or a combination of natural and synthetic fibres. There are a number of manufacturing techniques which may be used to manufacture the topsheet 29. For example, the topsheet 29 may be a nonwoven web of fibres spunbonded, carded, wet-laid, meltblown, hydroentangled, combinations of the above, or the like. An often used topsheet is carded and thermally bonded by means well known to those skilled in the fabrics art, for example made of staple length polypropylene fibres such as is manufactured by Veratec, Inc., a Division of International Paper Company, of Walpole, Massachusetts under the designation P-8.

The topsheet may be essentally attached throughout most or all of the surface of the absorbent core, or it may be only patially bonded thereto. The topsheet may comprise apertures to ease penetration of exudates therethrough, such as urine or feaces. The topsheet may further be fully or partially elasticated.

After having described the general aspects of absorbent articles, the specific features of the fixation on the wearer according to the present invention will be described.

First, the article or system requires a primary fastening system of the mechanical type, i.e essentially aiming at overcoming the gravitational forces and / or the frictional forces between the article and outside elements, such as the outer clothing of the wearer and /or the bedding elements by exploiting the frictional forces between the article and the skin of the wearer, or structural support forces such as registry with body contours such as hip bones of the wearer.

The absorbent article may alternatively be hold in place by conventional closure systems to fix the article around the waist of the wearer, such as by integral tape systems, which can comprise adhesive coated taped or mechanically engaging elements.

Thereby, the absorbent article is provided with a closure system comprising a tape tab fastening device and a landing member. The tape tab fastening device comprises an attachment area and a functional area, and the landing member is preferably a reinforcing strip or the alternative a portion of the backsheet. The functional elements may take on a number of configurations such as adhesive fastening elements, mechanical fastening elements, a combination of adhesive fastening elements and mechanical fastening elements, or any other means as are known to the man skilled in the art. A similar approach uses non-unitary elements, such as an attachable belt as described in EP-A-0.409.307 (Gipson et al.)

For these latter two elements with closure systems to encircle the waist of the wearer, the article is preferably further comprising elastication features operatively connected with these closure feature, so as to enhance and sustain the fit of the article during use.

Absorbent articles, which are sometimes referred to as "pant-style diapers" are unitary articles with a pant style design together with elastication features to allow fixation as the mentioned elastic pant, and also an absorbent core such as a panty comprising elasticated region and further described in EP-A-0.641.552 or US-A-5.246.433.

Alternatively , an absorbent article such as described in the above, may simply have a means for attaching it to the normal underwear of the user, such as often applied for light feminine hygiene articles, such as so-called "panty liner", and indicated in Fig. 1c by fixation means 30.

Whilst the fixation means as described so far are designed to be the unique fixation means, they often are a compromise between the first functionality of maintaining good contact between the body and the absorbent member and holding the weight of the article. It is obvious, that for example for a standing wearer, the fixation means has to hold the weight of heavy articles, especially when being used on incontinent adults.

Thus the non-leakage functionality of such articles is then depending to a large degree on the sealing means, such as elastication features, e.g. applied around the legs ("leg cuffs") or waist ("waistband").

Hence, in the present invention such primary mechanical fastening means is combined with a secondary fastening means of the topical adhesive attachment type, such as the unique fixation means for feminine hygiene articles as described in the background section.

Suitable adhesives have been described in EP 0850619 A and can be characterised by their visco-elastic properties, namely their elastic modulus abbreviated G'₃₇ and their viscous modulus G"₃₇, both at a temperature of 37°C (100° Fahrenheit) The adhesives have a dynamic elastic behaviour such that the difference Δ G'₃₇ of G'₃₇ as measured at a frequency of 100 rad/sec and G'₃₇ at a frequency of 1 rad/sec is not greater than 150 %, preferably 80 %, of G'₃₇ at 1 rad/sec or preferably less than 10000 Pa. The adhesives further have a dynamic viscous behaviour such that the difference Δ G"₃₇ of G"₃₇ at a frequency of 100 rad/sec and G"₃₇ at a frequency of 1 rad/sec is not greater than 10000 Pa.

The above rheological criteria are satisfied by adhesive compositions where the composition comprises from 51 % to 99.5 % by weight of a plasticising compound or composition which is liquid at 20°C, from 0.5 to 20 %, preferably 5 % to 15 % by weight of a polymeric compound or composition which is soluble or swellable in the plasticising compound or composition and with a tackifying resin in an amount in the range from 0 % to 600 % by weight of the polymeric compound. The plasticising compound or composition is preferably selected from the group consisting of water, alcohols, preferably glycerol, glycols, polyglycols, liquid polybutenes, oil or combinations thereof while the polymeric compound or composition is preferably selected from the group consisting of block-copolymer-thermoplastic-elastomers, styrene-block-copolymers and hydrogenated styrene-block-copolymers.

Quite generally the preferred body adhesive is at least partially hydrophobic, preferably 60 %, more preferably 80 %, by weight of the adhesive consist of hydrophobic components and most preferably none of the materials in the adhesive are hydrophilic, i.e. it is made totally from hydrophobic components.

According to the present invention, the forces required to support the loaded article on the wearer are split between two elements, thereby allowing to exploit the benefits of each of the individual systems.

For this aspect, there are three key design aspects to be balanced:

Firstly, the strength and force distribution aspects of the primary fixation means. The stronger the primary means holds the absorbent member, the stronger the risk of redmarking or skin irritation becomes. This becomes particularly relevant for articles with a relatively high ultimate storage capacity.

Secondly, the adhesion strength of the body adhesive to the skin. If this strength is too high, such as would be required support a loaded absorbent member, skin irritation can result.

Thirdly, the surface area, shape, and positioning of the body adhesive. If the body adhesive is applied so as to form a film on selected surface sections of the article, it only can be increased to certain limits, such as defined by available surface of the total article, which is not required for liquid penetration, and also by the body anatomy.

This is directly linked to positioning and shape of the body adhesive regions, so as to not design too much material into body creases and folds, and also to not position the adhesive regions to contact too much body hair.

In a further embodiment, the body adhesive is applied to the body side cuff end of the barrier cuff so as to seal effectively a possible gap between these cuffs and the body without excessive elastic forces.

In yet another execution of the present invention, the body adhesive is arranged such that it lies during use in the so called "low motion zones" of the wearer, thereby allowing maintenance of the fit even during use. For example the absorbent core can be provided with a front waist edge having an arcuate concave shape to fit below or at the abdominal crease of the wearer. Thereby the curve of the front waist edge approximates the curve of the abdominal crease of the wearer such that the absorbent core will naturally fit into the low motion zone to maximize the comfort for the wearer. The absorbent core can also be provided with arcuate concave side edges designed to fit in the leg creases of the wearer and to define a narrow crotch width which fits between the legs of the wearer. These leg cutouts are positioned farther forward in the absorbent core than the lateral centerline so that the front portion of the absorbent core is shorter in length to fit below the abdominal crease and to allow the absorbent core to fit higher over the buttocks and into the lumbar curve of the back. These zones are - exemplified for babies - described in more detail in PCT publication WO 94/28842 (LaVon et al.).

In a further execution, the adhesive can be applied in a specific pattern, such as can be achieved by printing of glues, or by stripes or other regular patterns, or by spraying the adhesive to the total of the surface or parts of the surface in a dot pattern, a spiral pattern or irregular fibrous pattern.

The adhesive can be applied to sections of the article which are firmly anchored and bonded to underlying elements, e.g. the topsheet to which the adhesive is applied can be bonded through to the underlying core or backsheet. Alternatively, the element to which the adhesive is applied to is not connected in the thickness direction to other elements, such that, for example the topsheet to which the body adhesive is applied to can - at least on certain sections of the article - detach therefrom. Thereby, void space might be created so as to ease handling of feaces, whilst the referred to opening in said topsheet still remains by means of the body adhesive in registry with body openings such as the anal opening.

In a second aspect of the present invention, the secondary attachment means eases application of the article.

Current absorbent articles or members having no body adhesive element are difficult to be applied in particular when considering adult incontinence products, where one nurse or caretaker works on elderly people, who have difficulties in moving, be this in a standing position of be this when lying in bed. Thus, today, fit of the absorbent article or member can be - and often is in reality - very poor.

In a second important aspect, the present invention provides significantly eased application of the article. This applies both for the situation with the user her- or himself apply the article or when another person such as a parent in case of baby diapers or a caretaker in case of disabled or handicapped elderly persons.

In these cases, the present invention allows to first apply the absorbent member without the need to tear or pull on the article or to stick various parts of the article to each other, but rather allow easy and also easily correctable application to the wearer.

This can be achieved with a relatively low adhesion force so as to not transmit excessive forces to the skin, and to not excessively strain the skin of the wearer upon removal of the article.

A further aspect of the present invention is concerned with the disposability of absorbent members. Whilst this is generally of no concern for absorbent articles which comprise fixation means of the fastening tape systems (and thereby especially when comprising adhesive tapes), insert pads such as widely used in adult incontinence field, often do not have any means to hold the absorbent member together after use. For such members, the secondary adhesive attachment means provides a useful and readily available closure means for rolled up absorbent members.

As has been laid out in the above, particularly suitable body adhesives must meet a balance of adhesion versus skin friendliness.

A particularly suitable adhesive has been prepared as follows: An oil based composition useful in the present invention was compounded using 9.9 % by weight of Kraton G-1651, a Styrene/Ethylene-Butylene/Styrene block copolymer containing 33% by weight styrene and available from Shell Co, and 59.3 % by weight of Kaydol, a paraffinic mineral oil available from Witco Co.

Moreover the composition contained 301 parts of tackifying resin per 100 parts of Kraton polymer. The tackifying resin was Escorez 5300, a hydrogenated resin available from Exxon Co.

Magnesium Stearate, available from Carlo Erba S.p.A., was used a a co-gelifying agent for oil at a level of 0.7 % by weight.

Irganox 1010, an antioxidant available from Ciba-Geigy, was added at a level of 0.3 % by weight.
So finally the formulation had the following percent composition:

| | |
|---|---|
| Kraton G-1651 | 9.9% by weight |
| Kaydol | 59.3 % by weight |
| Escorez 5300 | 29.8 % by weight |
| Magnesium Stearate | 0.7 % by weight |
| Irganox 1010 | 0.3% by weight |

The composition showed the following rheological properties at 37°C.
a) Elastic Modulus at 1 rad/s, G'₃₇ = 6876 Pa
b)
   - Ratio between Elastic and Viscous Modulus at 1 rad/s, G'₃₇ / G"₃₇ = 12.49
   - Ratio between Elastic and Viscous Modulus at 100 rad/s, G'₃₇ / G"₃₇ = 7.01
c) The ratio G'₃₇ at 100 rad/s over G'₃₇ at 1 rad/s was 1.308.

Of course, for such functionality not only the adhesive properties of the glue become relevant, but also the application of the glue on the article, such as where the glue is applied, and also how it is applied. Within this aspect, shape and size of the article is relevant, such as how the article is covering which body portions.

One important aspect is the area of the article, which is covered by body adhesive. Overall, less than half of the surface area should be covered in preferred executions between 1% and 40%, even more preferred executions between 2 % and 33 %.

As the body adhesives have a tendency to also stick to substances they are not supposed to stick to, these should be covered during manufacture and transport to the user, such as by other parts of the article from which these can be readily removed upon use, or by special covering means, which can be removed and possibly disposed before use, such as broadly used in the feminine hygiene field, wherein the adhesive is applied to the backsheet of the article and the covered with release paper.

A particular execution of an article according to the present invention has been prepared by modifying a commercially available severe adult incontinence product, which is sold under the trade name "DAISY DEO MAXI SUPER by Procter & Gamble throughout various Scandinavian countries. The product has an ultimate storage capacity of about 667 ml per pad. Such a product is designed to be worn in combination with a net pant as external, primary fixation means, such as also sold by Procter & Gamble in various Scandinavian countries under the trade name DAISY PANT.

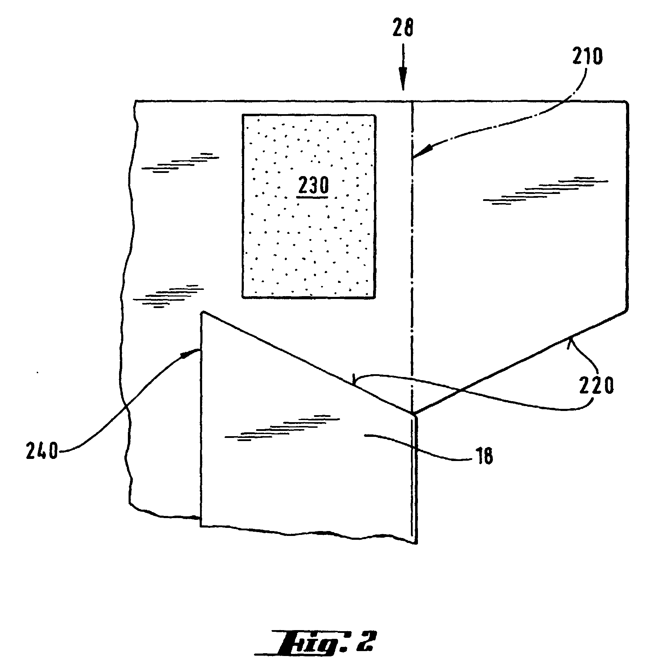

This product has been modified by applying an adhesive as described above at a basis weight of between 1000 gsm and 1350 gsm at the locations as indicated in Fig. 2, thereby replacing partly conventional adhesives for forming the tubular arrangement 22,23 in Fig. 1. Thereby, the adhesive is covered by the folded edges flaps, ,thus providing an further benefit of allowing easy transport and handling before application of the product without requiring additional covering of the adhesive such as with release paper. The patches were made out of stripes of adhesive having a width of 2 cm and a length of 6 cm, positioned at the coners 25 to 28 of the article, such as indicated for comer 28 in Fig. 2. Therein, the lateral edge 18, which is folded inwardly along the fold line 210 is folded outwardly in the end region of the article by means of a cut 220 through the edge.

The part which remains inwardly folded is permanently attached in the region 240 whereas the outwardly folded part is refolded onto the body adhesive patch 230.

The total area of coverage with body adhesive is thus about 48 cm² or about 4% of the total surface.

Without wishing to be bound by the theory, it is believed, that absorbent articles comprising the features of the present invention synergistically exploit the benefits of primary and secondary fixations means, as both of them do provide a certain support of the weight of the article, but the primary fixation means has some force elements reducing the tendency of the article to peel away during use, and thus allow the shear forces to dominate. At the same time, these shear forces minimise the effect which (even small) movements of the wearer have of fit deterioration, in particular in combination with the gravity forces, which often results in what is generally referred to as "sagging". For example, the elastic pant provides sufficient pressure to support the adhesion of the body adhesive on the skin of the wearer, however does not need to provide all the pressure forces so as to maintain the article exactly in place.

Of course, there are elements which make the design of the absorbent articles as described particularly robust, e.g. designing the adhesive such that the forces that pull it down in use act on the bond between the adhesive and the skin of the wearer as shear or pull forces and not as peel forces. Further, the forces as transmitted between skin and adhesive can be further transmitted to the rest of the article, such as by having all layers of the article bonded to each other in the areas where the adhesive is applied to.

In an alternative design, the body adhesive can be applied to certain elements of the article, which then might not be firmly bonded to the underlying elements. This can be in a non-limiting way be exemplified by looking at spray application of the body adhesive to the topsheet of the article, preferably in specific areas of the article, where the topsheet is not attached to the underlying structure, and is designed to allow penetration of faeces therethrough such as through an opening therein. Then, the body adhesive can enhance the contact of the topsheet with the skin and therby better function as means for separating feaces from the skin of the wearer.

In alternative designs for articles, the body adhesive can be applied in other forms, that the above described full coverage in certain areas. For example, the adhesive can be applied in stripes or beads, by spraying in a spiral form, or droplets.

Also, when the article is applied by a different person than the wearer, such as a parent in case for babies, or care taker in case of adult incontinence articles, the body adhesive allows significantly eased application by enabling accurate fixation before application of the pant. Also, the article can be positioned accurately with one end first, then the wearer can be turned or moved to allow easy access of the other body portions (e.g. the back) and the remainder of the article can be attached.

Or in case of a diaper comprising a tape closure system, the body adhesive allows accurate positioning followed by easy activation of the closure system even when the wearer is moving (such as is often the case for babies) or the wearer is difficult or heavy to move (such as is the case with adults).

It is further believed, that the present invention provides particular benefits for situations which have special requirements for gentle treatment of the skin of the wearer, as it provides less mechanical stress for the skin than pure mechanical affixation or pure adhesive affixation each on their own. This is particularly true for skin of elderly people, and/or if absorbent articles are permanently worn.

## Claims

1. Absorbent article comprising a fixation means of the body contact adhesive type (230) comprising adhesive compositions where the composition comprises from 51 % to 99.5 % by weight of a plasticising compound or composition which is liquid at 20°C, from 0.5 to 20 %, preferably 5 % to 15 % by weight of a polymeric compound or composition, the polymeric compound or composition being soluble or swellable in the plasticising compound or composition **characterized in that** the absorbent article further comprises mechanical fixation means (30).

2. Absorbent article according to claim 1 further **characterised in that** body contact adhesive (230) is applied to body oriented surface of said article.

3. Absorbent article according to any of claims 1 to 2 further **characterised in that** the body adhesive (230) covers less than 50% of the surface of the article.

4. Absorbent article according to claim 3, wherein the body adhesive (230) covers between 1% of the surface area of the article to 40%.

5. Absorbent article according to claim 4 wherein the body adhesive (230) covers between 2 and 33% of the surface area of the article.

6. Absorbent article according to any of claims 1 to 5 further **characterised in that** the body adhesive (230) is applied in a pattern made of lines, dots, stripes, spiral or melt-blown spray.

7. Absorbent article according to any of claims 1 to 6 further **characterised in that** the article has a storage capacity of at least 70 ml.

8. Absorbent article according to any of claims 1 to 6 further **characterised in that** the article has a storage capacity of at least 90 ml.

9. Absorbent article according to any of claims 1 to 4 further **characterised in that** the article has a design capacity of at least 165 ml.

10. Absorbent article according to any of the preceding claims wherein said adhesive is a composition of materials comprising:
- from 51% to 99.5% by weight of a plasticising compound or composition which is liquid at 20°C;
- from 0.5% to 20% by weight of a polymeric compound or composition which is solvable or swellable in said plasticising compound or composition;
- a tackifying resin in an amount of from 0% to 600% by weight of said polymeric compound or composition.

11. Absorbent article according to any of the preceding claims, wherein in the primary fixation means is integral with the absorbent body.

12. Absorbent article according to any of the preceding claims, wherein said primary fixation means comprises tape closure system.

13. Absorbent article according to any of the preceding claims, wherein said tape closure system comprised adhesive closure means.

14. Absorbent article according to any of the preceding claims, wherein said closure system comprises mechanically engaging closure means.

15. Absorbent article according to any of claims 1 to 9, wherein said primary fixation means comprises elastic features for encircling the waist of the wearer so as to provide mechanical fixation by providing friction forces between the article and the wearer.

16. Absorbent article according to claim 15 wherein said primary fixation means is a stretch pant.

17. Absorbent article according to any of the preceding claims wherein the adhesive is applied to fit on the low motion zones of the wearer.

18. Absorbent article according to any of the preceding claims, wherein the adhesive is applied such that it allows ease of disposability after use.

19. Absorbent article according to any of the preceding claims wherein the topsheet comprises an aperture for allowing faeces to pass through, and regions comprising bodyadhesive.

## Patentansprüche

1. Absorbierender Artikel umfassend Befestigungsmittel des Körperkontakt-Klebetyps (230) umfassend Klebstoffzusammensetzungen, wobei die Zusammensetzung umfasst von 51 Gew.-% bis 99 Gew.-% einer plastifizierenden Verbindung oder Zusammensetzung, die bei 20° C flüssig ist, 0,5 bis 20 Gew.-%, vorzugsweise 5 Gew.-% bis 15 Gew.-% einer Polymerverbindung oder -zusammensetzung, wobei die Polymerverbindung oder -zusammensetzung in der plastifizierenden Verbindung oder Zusammensetzung lösbar oder quellbar ist, **dadurch gekennzeichnet, dass** der absorbierende Artikel ferner mechanische Befestigungsmittel (30) umfasst.

2. Absorbierender Artikel nach Anspruch 1, ferner **dadurch gekennzeichnet, dass** der Körperkontakt-Klebstoff (230) auf eine dem Körper zugewandte Oberfläche des Artikels aufgebracht ist.

3. Absorbierender Artikel nach einem der Ansprüche 1 bis 2, ferner **dadurch gekennzeichnet, dass** der Körperklebstoff (230) weniger als 50 % der Oberfläche des Artikels bedeckt.

4. Absorbierender Artikel gemäß Anspruch 3, wobei der Körperklebstoff (230) zwischen 1 % und 40 % des Oberflächenbereiches des Artikels bedeckt.

5. Absorbierender Artikel gemäß Anspruch 4, wobei der Körperklebstoff (230) zwischen 2 und 33 % des Oberflächenbereiches des Artikels bedeckt.

6. Absorbierender Artikel gemäß einem der Ansprüche 1 bis 5, ferner **dadurch gekennzeichnet, dass** der Körperklebstoff (230) in einem Muster aufgebracht ist, dass aus Linien, Punkten, Streifen, spiralförmig oder schmelzgeblasenem Spray aufgebracht ist.

7. Absorbierender Artikel nach einem der Ansprüche 1 bis 6, ferner **dadurch gekennzeichnet, dass** der Artikel eine Speicherkapazität von zumindest 70 ml aufweist.

8. Absorbierender Artikel nach einem der Ansprüche 1 bis 6, ferner **dadurch gekennzeichnet, dass** der Artikel eine Speicherkapazität von zumindest 90 ml aufweist.

9. Absorbierender Artikel gemäß einem der Ansprüche 1 bis 4, ferner **dadurch gekennzeichnet, dass** der Artikel eine spezifizierte Kapazität von zumindest 165 ml aufweist.

10. Absorbierender Artikel nach einem der vorgenannten Ansprüche, wobei der Klebstoff eine Zusammensetzung von Materialien ist, die umfassen:
- von 51 Gew.-% bis 99,5 Gew.-% einer plastifizierenden Verbindung oder Zusammensetzung, die bei 20° C flüssig ist;
- von 0,5 Gew.-% bis 20 Gew.-% einer Polymerverbindung oder
- zusammensetzung, die in der plastifizierenden Verbindung oder
- zusammensetzung lösbar oder quellbar ist;
- ein klebrigmachendes Harz in einer Menge von 0 Gew.-% bis 600 Gew.-% der Polymerverbindung oder -zusammensetzung.

11. Absorbierender Artikel gemäß einem der vorgenannten Ansprüche, wobei die primäre Fixiereinrichtung integral mit dem absorbierenden Körper ist.

12. Absorbierender Artikel nach einem der vorgenannten Ansprüche, wobei die primäre Fixiereinrichtung ein Bandverschlusssystem umfasst.

13. Absorbierender Artikel nach einem der vorgenannten Ansprüche, wobei das Bandverschlusssystem Klebeverschlussmittel umfasst.

14. Absorbierender Artikel nach einem der vorgenannten Ansprüche, wobei das Verschlusssystem mechanisch eingreifende Verschlussmittel umfasst.

15. Absorbierender Artikel nach einem der Ansprüche 1 bis 9, wobei die primäre Fixiereinrichtung elastische Eigenschaften zum Umschließen der Hüfte des Trägers umfasst, um so für eine mechanische Fixierung zu sorgen, indem Reibungskräfte zwischen dem Artikel und dem Träger geschaffen werden.

16. Absorbierender Artikel gemäß Anspruch 15, wobei die primäre Fixiereinrichtung eine Stretchhose ist.

17. Absorbierender Artikel nach einem der vorgenannten Ansprüche, wobei der Klebstoff so aufgebracht ist, dass er auf die wenig Bewegung zeigenden Bereiche des Trägers passt.

18. Absorbierender Artikel nach einem der vorgenannten Ansprüche, wobei der Klebstoff so aufgebracht ist, dass er eine einfache Wegwerfbarkeit nach Gebrauch erlaubt.

19. Absorbierender Artikel nach einem der vorgenannten Ansprüche, wobei die Decklage aufweist eine Öffnung, um Körperausscheidungen zu erlauben, hindurchzutreten, und Bereiche, die Körperklebstoff umfassen.

## Revendications

1. Article absorbant comprenant un moyen de fixation du type adhésif (230) au contact du corps, comprenant des compositions adhésives où la composition renferme de 51 % à 99,5 % en poids d'un composé plastifiant ou d'une composition plastifiante qui est liquide à 20° C, de 0,5 à 20 %, de préférence de 5 % à 15 % en poids d'un composé ou d'une composition polymère, le composé ou la composition polymère étant soluble ou gonflable dans le composé plastifiant ou la composition plastifiante, **caractérisé en ce que** l'article absorbant comprend, en outre, un moyen de fixation mécanique (30).

2. Article absorbant selon la revendication 1, **caractérisé, en outre, en ce que** l'adhésif (230) de contact avec le corps est appliqué à une surface orientée vers le corps dudit article.

3. Article absorbant selon l'une quelconque des revendications 1 ou 2, **caractérisé, en outre, en ce que** l'adhésif (230) de contact avec le corps couvre moins de 50 % de la surface de l'article.

4. Article absorbant selon la revendication 3, dans lequel l'adhésif (230) de contact avec le corps couvre entre 1 % de la superficie de l'article et 40 %.

5. Article absorbant selon la revendication 4, dans lequel l'adhésif (230) de contact avec le corps couvre entre 2 et 33 % de la superficie de l'article.

6. Article absorbant selon l'une quelconque des revendications 1 à 5, **caractérisé, en outre, en ce que** l'adhésif (230) de contact avec le corps est appliqué selon un motif constitué de lignes, de points, de bandes, de spirales ou une projection soufflée à l'état fondu.

7. Article absorbant selon l'une quelconque des revendications 1 à 6, **caractérisé, en outre, en ce que** l'article a une capacité de stockage d'au moins 70 ml.

8. Article absorbant selon l'une quelconque des revendications 1 à 6, **caractérisé, en outre, en ce que** l'article a une capacité de stockage d'au moins 90 ml.

9. Article absorbant selon l'une quelconque des revendications 1 à 4, **caractérisé, en outre, en ce que** l'article a une capacité de stockage d'au moins 165 ml.

10. Article absorbant selon l'une quelconque des revendications précédentes, dans lequel ledit adhésif est une composition de matériaux comprenant :
- de 51 % à 99,5 % en poids d'un composé plastifiant ou d'une composition plastifiante qui est liquide à 20° C ;
- de 0,5 % à 20 % en poids d'un composé ou d'une composition polymère qui est soluble ou gonflable dans ledit composé plastifiant ou ladite composition plastifiante ;
- une résine d'adhésivité à raison de 0 % à 600 % en poids dudit composé ou composition polymère.

11. Article absorbant selon l'une quelconque des revendications précédentes, dans lequel le dispositif de fixation primaire est solidaire du corps absorbant.

12. Article absorbant selon l'une quelconque des revendications précédentes, dans lequel ledit moyen de fixation primaire comprend un système de fermeture à ruban.

13. Article absorbant selon l'une quelconque des revendications précédentes, dans lequel ledit système de fermeture à ruban comprend un moyen de fermeture adhésif.

14. Article absorbant selon l'une quelconque des revendications précédentes, dans lequel ledit système de fermeture comprend un moyen de fermeture à prise mécanique.

15. Article absorbant selon l'une quelconque des revendications 1 à 9, dans lequel ledit moyen de fixation primaire comprend des éléments élastiques pour entourer la taille de l'utilisateur afin d'avoir une fixation mécanique en fournissant des forces de frottement entre l'article et l'utilisateur.

16. Article absorbant selon la revendication 15, dans lequel ledit moyen de fixation primaire est une culotte étirée.

17. Article absorbant selon l'une quelconque des revendications précédentes, dans lequel l'adhésif est appliqué pour s'adapter sur les zones de faible mouvement de l'utilisateur.

18. Article absorbant selon l'une quelconque des revendications précédentes, dans lequel l'adhésif est appliqué afin de permettre une facilité de mise au rebut après l'utilisation.

19. Article absorbant selon l'une quelconque des revendications précédentes, dans lequel la feuille de dessus comprend une ouverture pour permettre le passage des matières fécales et des régions comprenant un adhésif de contact avec le corps.
